# EUROPEAN PATENT SPECIFICATION

(11) **EP 4 321 175 B1**
(45) Date of publication and mention of the grant of the patent: **16.07.2025**
(21) Application number: 22461593.0
(22) Date of filing: 11.08.2022
(51) Int. Cl.: A61K 41/00, A61K 47/69, A61K 9/00, A61K 9/51, A61K 31/337, A61K 31/517, A61K 31/565, A61P 35/00

(54) **MAGNETIC POLYMER NANOCAPSULES SUITABLE FOR USE IN THERAPY, ESPECIALLY ANTI-CANCER ONES**
MAGNETISCHE POLYMERNANOKAPSELN, DIE ZUR VERWENDUNG IN DER THERAPIE, INSBESONDERE IN DER ANTIKREBSTHERAPIE, GEIGNET SIND
NANOCAPSULES POLYMÈRES MAGNÉTIQUES APPROPRIÉES POUR UNE UTILISATION EN THÉRAPIE, EN PARTICULIER EN THÉRAPIE ANTICANCÉREUSE

(43) Date of publication of application: 14.02.2024
(73) Proprietor: CHDE Polska S.A., 35-304 Rzeszów (PL)
(72) Inventor: Zapotoczny, Szczepan, 30-381 Kraków (PL); Odrobinska-Balis, Joanna, 30-611 Kraków (PL); Gumieniczek-Chlopek, Elzbieta, 32-050 Skawina (PL); Kapusta, Czeslaw, 31-355 Kraków (PL)
(74) Representative: Matyka, Malgorzata

(56) References cited:
- WO-A1-2017/014655
- PODGÓRNA KAROLINA ET AL: "Synthesis of polyelectrolyte nanocapsules with iron oxide (Fe3O4) nanoparticles for magnetic targeting", COLLOIDS AND SURFACES A : PHYSIOCHEMICAL AND ENGINEERINGS ASPECTS, ELSEVIER, AMSTERDAM, NL, vol. 505, 12 February 2016 (2016-02-12), pages 132 - 137, XP029640933, ISSN: 0927-7757, DOI: 10.1016/J.COLSURFA.2016.02.017
- GUMIENICZEK-CHLOPEK ELZBIETA ET AL: "Hydrophobically Coated Superparamagnetic Iron Oxides Nanoparticles Incorporated into Polymer-Based Nanocapsules Dispersed in Water", MATERIALS, vol. 13, no. 5, 9 March 2020 (2020-03-09), CH, pages 1219, XP093017638, ISSN: 1996-1944, DOI: 10.3390/ma13051219

## Description

The invention relates to magnetic polymer nanocapsules and their use in anti-cancer therapy, the nanocapsules being core-shell polymer nanocapsules containing nanoparticles made of iron oxides that carry hydrophobic active substances.

Core-shell magnetic nanocapsules containing iron oxide nanoparticles (wüstite-maghemite) are known from a research publication of the authors: E. Gumieniczek-Ch 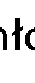 opek, J. Odrobinska, T. Straczek, A. Radziszewska, S. Zapotoczny, C. Kapusta entitled "Hydrophobically Coated Superparamagnetic Iron Oxides Nanoparticles Incorporated into Polymer-Based Nanocapsules Dispersed in Water" Materials 2020, 13, 1219, where such systems, due to the use of biocompatible components, can be tested in biological environments for magnetically-controlled delivery of active hydrophobic substances.

Polish patent description PL229276B1 disclosed biocompatible polysaccharide capsules based on liquid oil cores with diameters not exceeding 1 µm, stabilized without the need to use low-molecular surfactants, characterized by effective encapsulation of hydrophobic compounds and high stability in aqueous suspension.

Polish patent application P.425141 disclosed a nanocapsule containing a liquid oil core made of oleic acid and a stabilizing shell made of hydrophobically-modified hyaluronic acid. The document further covers the use of the subject oil core nanocapsule in anti-cancer therapy, particularly in the treatment of mammary gland cancer or melanoma.

US patent application US2016199308A1 disclosed magnetic capsules with aqueous nanocore and a drug delivery shell, containing a plurality of amphiphilic proteins (where the amphiphilic protein has a hydrophilic chain-end and a hydrophobic chain-end), a plurality of iron oxide nanoparticles (Fe₃O₄), a hydrophilic drug and a hydrophobic drug. The shelled magnetic nanocore capsules of the invention have high drug loading capacity, the ability to encapsulate hydrophobic and hydrophilic pharmaceuticals simultaneously, and therefore can be used for targeted drug delivery, magnetic resonance imaging and hyperthermia.

K. Podgórna and K. Szczepanowicz, "Synthesis of polyelectrolyte nanocapsules with iron oxide (Fe3O4) nanoparticles for magnetic targeting", Colloids and Surfaces A: Physicochemical and Engineering Aspects, 2016, Volume 505, 132-137, disclose magnetic core/shell polymer nanocapsules comprising a liquid core comprising magnetic iron oxide nanoparticles and a multilayer shell comprising cationic poly-L-lysine and anionic poly-L-glutamic acid.

Numerous scientific groups focus their research on finding a solution that allows the delivery of the active substance to the body in a controlled manner, while maintaining the concentration corresponding to the therapeutic dose and omitting the side effects associated with damage to healthy cells. Among the various systems dedicated to this type of application, a large group of systems made of polymeric materials should be distinguished. One example of such carriers are capsules based on liquid oil cores. The method of creating such systems uses the emulsification method, which consists in suspending oil droplets in an aqueous solution with an appropriate pH. Due to thermodynamic instability, the dispersed oil droplets forming the core of the capsule require the use of an appropriate stabilizer. In the case of biomedical applications, it is right to abandon the use of low molecular weight surfactants for the stabilization of compounds and to use properly grafted biopolymers in the form of e.g. modified hyaluronic acid or chitosan instead. Due to their amphiphilic nature, the compounds used in the aqueous environment are arranged with their hydrophilic main chain on the surface of the oil droplets, while the side hydrophobic groups penetrate the interior of the carrier, forming closed structures of the oil core/polymer shell type. Moreover, the presence of a charge on the backbone of the compound used to stabilize the carrier enables the surface charge of the entire system to be controlled by the use of the method of alternating application of oppositely charged layers of polyelectrolytes (LbL, Layer by Layer).

The systems obtained in this way show high stability, appropriate size and the ability to encapsulate hydrophobic compounds [J. Szafraniec et al., Nanoscale, 2015, 7, 5525-5536]. In order to better target the delivery and release process of the encapsulated substance in the carriers presented above, it is possible to enrich the systems with magnetic properties by placing magnetic nanoparticles in their structure. Due to the biomedical application, nanoparticles of iron oxides are used for this purpose, the hydrophobic coating of which enables placing the nanostructures in the oil core of the carrier. Very important, strong magnetic properties enabling to control the entire system are achieved by the superparamagnetic nature of the nanoparticles used [J. Odrobińska et al., ACS Appl. Mater. Interfaces 2019, 11, 10905-10913]. Among the products available on the medical market, there are several systems that use magnetic nanoparticles, but at the moment none of them is intended for magnetically navigated, targeted and controlled delivery of active substances [D.D. Stueber et al., Pharmaceutics 2021, 13, 943]. Very stable nanocapsules based on liquid oil cores stabilized with appropriately modified biopolymers are well known in the literature, and magnetically-controlled systems designed for targeted and controlled delivery of active substances have been also presented. However, the combination of these two solutions that have been used in the present invention is not available.

Numerous methods using magnetic nanoparticles and polymer nanocapsules can be found in the current state of the art, yet the development and implementation of an effective and efficient anticancer therapy is still a challenge for modern science. In the state of the art, the lack of polymer nanocapsules carrying active substances with high stability and magnetic properties for use in the treatment, in particular of breast cancer, is a huge problem in anti-cancer therapies. Moreover, low efficiency of hydrophobic active substance encapsulation results in an insufficient concentration of the active substance in polymer capsules and released in cancer cells, corresponding to the therapeutic dose. Another issue is few targeted therapies in the prior, since it is impossible to control (selective targeting/navigation) polymeric nanocapsules carrying active substances to malignant cells, especially breast cancer cells, using a static magnetic field. Another problem is that the release of hydrophobic active substances in cancer cells using an alternating magnetic field is impossible, as well as that the side effects of currently used therapeutics in anti-cancer therapy cannot be ignored, while widely used chemotherapies have highly negative effects on the human body. In the already known solutions using polymer capsules, the relatively large dimensions of the core-shell systems hinders penetration of the polymer capsules containing active substances into cancer cells, and the frequent occurrence of cytotoxicity of polymer capsules to normal cells in the body could not be completely eliminated. An additional problem is the inability to perform targeted and controlled anti-cancer therapy with the use of magnetic polymer capsules carrying active substances.

The object of the present invention was to provide polymer nanocapsules carrying active substances, characterized by very high stability and magnetic properties, as well as high efficiency of encapsulation of hydrophobic active substances, which allows achieving a therapeutic concentration of the active substance encapsulated in the polymer capsules and released in cancer cells. Another goal was to enable navigation (selectively targeting) the polymer capsules carrying active substances to cancer cells, especially breast cancer cells, with the use of a static magnetic field and providing the possibility of hydrophobic active substance release in cancer cells with the use of an alternating magnetic field. Another object of the invention was to completely eliminate the side effects of therapy with magnetic, polymer nanocapsules used so far in anti-cancer therapy. Another object of the present invention was to obtain very small size (nanometric size) of the core-shell systems (capsules) transporting active substances, that would easily penetrate into cancer cells and would show no cytotoxicity to normal cells in the body.

Another object of the present invention was to develop an efficient, effective anti-cancer targeted therapy with the possibility of fully controlling the release of active substances with the use of magnetic polymer capsules carrying these substances.

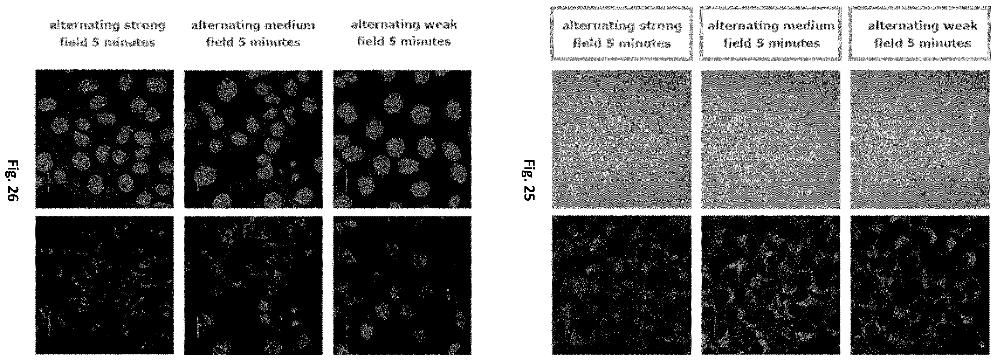

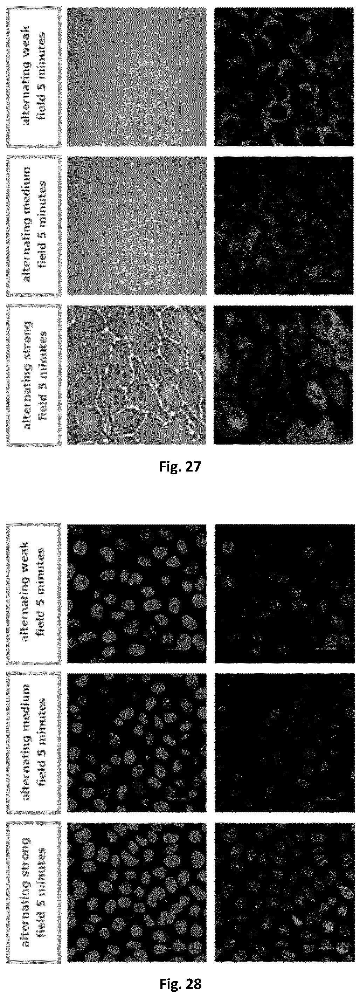

Unexpectedly, all the above-mentioned technical problems have been solved by the present invention. The subject of the invention is: a nanocapsule as defined in the appended claims and its medical use. The nanocapsules prepared according to the invention are able to cross the cell membrane barrier, magnetically assisted, without damaging the structure of the carrier (nanocapsules). In the course of the research that led to the present invention, it was found that the negative surface charge of capsules is necessary for the effective crossing of capsules through the cell membranes into cells and for the release of the capsule contents throughout the cell volume; see Fig. 25 (positively charged capsules) and Fig. 27 (negatively charged capsules) The positively charged capsules concentrate more around or stay within the cell membrane rather than they pass through.

At the same time, it has been observed that the application of an alternating magnetic field of an appropriate intensity leads to a rapid and complete release of the contents of the nanocapsules, prepared according to the present invention, into the cells.

The invention is the use of magnetically-controlled polymer capsules based on liquid oil cores, which in their structure contain magnetic iron oxide nanoparticles and transported active substances. The presented carriers are spherical core-shell systems with dimensions of the order of several hundred nanometers. These systems show high stability, enable effective encapsulation of hydrophobic active substances and are capable of magnetically controlled (static magnetic field) delivery and release of the transported cargo (effect of an alternating magnetic field). The carrier gains magnetic properties by placing spherical nanoparticles in the hydrophobic environment of the core, with dimensions not larger than 30 nanometers, composed of iron oxides and having an outer lipophilic coating. Due to their surface made of biocompatible polysaccharides, the polymer capsules do not exhibit cytotoxicity and can be absorbed by cells, and their contents can be released inside the cells in effect of an alternating magnetic field.

The subject of the invention in the exemplary embodiments is shown in the drawing, in which:
Fig. 1 shows the temperature conditions as a function of time during the thermal reaction of iron (III) oleate decomposition,
Fig. 2 shows a STEM image of nanoparticles obtained by thermal decomposition of an organometallic precursor,
Fig. 3 shows the FT-IR spectrum of magnetic nanoparticles and oleic acid,
Fig. 4 shows X-ray diffraction patterns of magnetic nanoparticles, microcrystalline magnetite and wüstite, with Miller indices marked,

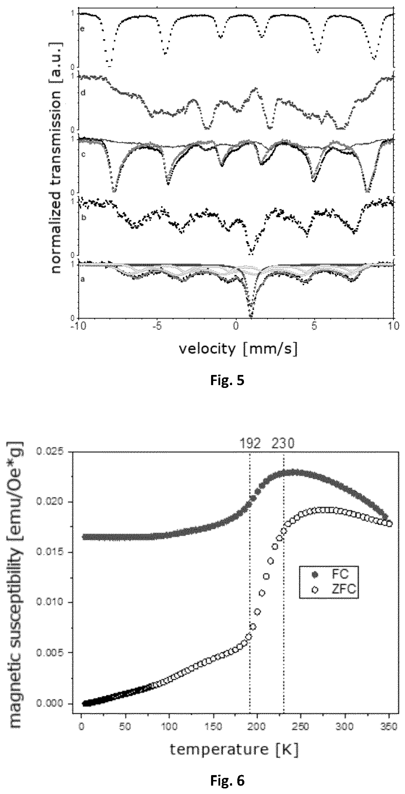

Fig. 5 shows the spectrum of magnetic nanoparticles obtained by Mössbauer spectroscopy (black points indicate the measurement results): a) measurement made at room temperature with fitted wüstite sextet (blue line), magnetite sextets (blue and green lines) and the total fit (red line); b) measurement of magnetic nanoparticles at room temperature 8 months after their synthesis; c) measurement at liquid nitrogen temperature including the wüstite content (blue points) and excluding the wüstite phase (red points); d) microcrystalline wüstite spectrum measured at liquid nitrogen temperature; e) microcrystalline maghemite spectrum measured at liquid nitrogen temperature,
Fig. 6 shows the magnetic susceptibility of magnetic nanoparticles as a function of temperature, measured in the field of 100 Oe,
Fig. 7 shows the magnetization curves of magnetic nanoparticles as a function of the applied field, measured over a temperature range of 4-300 K,
Fig. 8 shows the changes in the hydrodynamic diameter value and the zeta potentials of the magnetic cationic capsules measured over 48 weeks,
Fig. 9 shows a cryo-TEM image of magnetic cationic capsules based on oil cores,
Fig. 10 is a schematic diagram of an anionic capsule based on an oil core with encapsulated magnetic nanoparticles,
Fig. 11 shows the changes in the hydrodynamic diameter value and the zeta potentials of the magnetic anionic capsules measured over 48 weeks,
Fig. 12 shows images of magnetic capsules taken with a confocal microscope: A) cationic capsules (image taken on the day the capsules were prepared); B) cationic capsules (image taken after 2 weeks of storage of the capsules); C) anionic capsules (image taken on the day the capsules were prepared); D) anionic capsules (image taken after 2 weeks of storage of the capsules). Scale: 1 µm,
Fig. 13 shows the magnetization of cationic magnetic capsules as a function of an applied field at a temperature of 300 K,
Fig. 14 shows the layout of a 24-well plate prepared to determine capsule toxicity to 4T1 cell line cells,
Fig. 15 is a cell survival graph of the 4T1 cell line cells (determined by the XTT assay) in contact with cationic magnetic capsules at various concentrations,
Fig. 16 is a cell survival graph of the 4T1 cell line cells (determined by the XTT assay) in contact with anionic magnetic capsules at various concentrations,
Fig. 17 is a schematic diagram of an experiment of pulling cells with a static magnetic field,
Fig. 18 shows confocal microscopic images of cells stained with Hoechst (left column, DAPI filter) and propidium iodide (right column, TRICT filter) fluorescent dyes after removal from the incubator and subjected to 5- and 15-minute static magnetic field exposure. Scale: 20 µm,
Fig. 19 shows confocal microscopic images of cells subjected to an experiment of introducing fluorescently-labelled magnetic cationic capsules with a static external magnetic field. Left column: transmitted light and TRITC filter, right column: TRITC filter. Scale: 20 µm,
Fig. 20 shows confocal microscopic images of cells stained with Hoechst (left column, DAPI filter) and propidium iodide (right column, TRICT filter) fluorescent dyes after subjecting to an experiment of introducing magnetic cationic capsules with a static external magnetic field. Scale: 20 µm.
Fig. 21 shows confocal microscopic images of cells subjected to an experiment of introducing fluorescently-labelled magnetic anionic capsules with a static external magnetic field. Left column: transmitted light and TRITC filter, right column: TRITC filter. Scale: 20 µm,
Fig. 22 shows confocal microscopic images of cells stained with Hoechst (left column, DAPI filter) and propidium iodide (right column, TRICT filter) fluorescent dyes after subjecting to an experiment of introducing magnetic anionic capsules with a static external magnetic field. Scale: 20 µm,
Fig. 23 shows a schematic diagram of an experiment of the encapsulated substance release inside the cells with an external alternating magnetic field,
Fig. 24 is a confocal microscope image of cells stained with Hoechst (left column, DAPI filter) and propidium iodide (right column, TRICT filter) fluorescent dyes subjected to a 15-minute static magnetic field exposure followed by an external alternating magnetic field exposure. Scale: 20 µm,
Fig. 25 is a confocal microscope image of cells subjected to an experiment of introducing and release of substance encapsulated in fluorescently-labelled magnetic cationic capsules with a 15-minute static external magnetic field exposure followed by a relevant alternating magnetic field exposure. Left column: transmitted light and TRITC filter, right column: TRITC filter. Scale: 20 µm,
Fig. 26 shows confocal microscopic images of cells stained with Hoechst (left column, DAPI filter) and propidium iodide (right column, TRICT filter) fluorescent dyes subjected to an experiment of introducing and release of substance encapsulated in magnetic cationic capsules with a 15-minute static external magnetic field exposure followed by a relevant alternating magnetic field exposure. Scale: 20 µm,
Fig. 27 is a confocal microscope image of cells subjected to an experiment of introducing and release of substance encapsulated in fluorescently-labelled magnetic anionic capsules with a 15-minute static external magnetic field exposure followed by a relevant alternating magnetic field exposure. Left column: transmitted light and TRITC filter, right column: TRITC filter. Scale: 20 µm,
Fig. 28 shows confocal microscopic images of cells stained with Hoechst (left column, DAPI filter) and propidium iodide (right column, TRICT filter) fluorescent dyes subjected to an experiment of introducing and release of substance encapsulated in magnetic anionic capsules with a 15-minute static external magnetic field exposure followed by a relevant alternating magnetic field exposure. Scale: 20 µm.

### Example 1. High-temperature synthesis of iron oxide nanoparticles with a hydrophobic coating.

Magnetic nanoparticles placed in the capsule structure are supposed to impart magnetic properties to the entire carrier. These nanostructures were obtained through a two-step synthesis involving thermal decomposition of a previously synthesized organometallic precursor in the presence of oleic acid. The application of this method allows to obtain nanoparticles with a narrow size distribution, consisting of iron oxides and showing strong magnetic properties. The presence of a hydrophobic substance during the synthesis is to provide adequate coverage of the structures, allowing them to be placed in the oil core of the capsule.

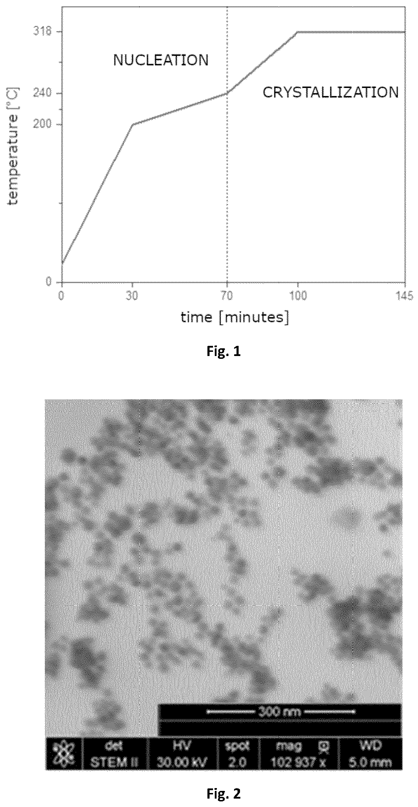

The synthesis of iron (III) oleate was performed based on the procedure previously presented by Leszczyński et al. [Leszczyński, B. *et al.* The influence of oxidation process on exchange bias in eggshaped FeO/Fe₃O₄ core/shell nanoparticles. *J Magn Magn Mater* **416,** 269-274 (2016)]. To obtain the organometallic precursor, 30 mL of deionized water, 40 mL of ethanol, 70 mL of hexane, 3.25 g of anhydrous iron (III) chloride, and 18.25 g of sodium oleate were mixed together. The resulting solution was heated to 60°C and kept under constant stirring for 5 hours. The dark hydrophobic portion was then separated off, rinsed with deionized water and heated to 40°C to evaporate residual hexane. The synthesis leading directly to nanoparticle generation was carried out according to the procedure previously described by Park et al. [Park J. et al. Ultra-large-scale syntheses of monodisperse nanocrystals Nat Mater 3, 891-895 (2004)]. Initially, 42 mL of octadecane, 1.1 mL of oleic acid and 7 g of the previously prepared iron (III) oleate were mixed. The entire reaction was carried out under inert gas conditions, with constant stirring, and under the temperature regime shown in the diagram below (Fig. 1). The resulting product was cooled to room temperature, washed several times with ethanol, and unreacted substrates were removed by sonication in hexane (continuous, 5 minutes) and centrifugation (5,000 rpm, 5 minutes). The obtained nanoparticles were vacuum dried.

### Physicochemical characteristics of the nanoparticles

Imaging with scanning electron transmission microscopy (STEM) (Fig. 2) confirmed the spherical shape of individual nanoparticles, while the analysis of the obtained image indicated an average size of the structures of 15 nm, with a narrow distribution of this parameter.

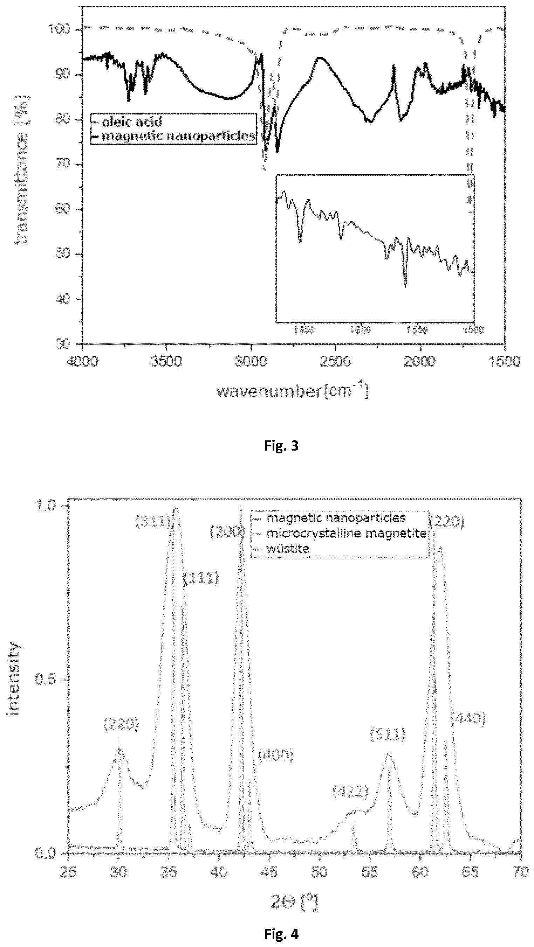

Selection of the synthesis method of the magnetic nanoparticles was strictly conditioned by the possibility of obtaining structures with appropriate coating enabling them to be placed in the hydrophobic interior of the capsule. In order to determine the correct deposition of the oleic acid layer on the nanoparticle surface, Fourier infrared spectroscopy (FT-IR) measurements and TGA-DSC thermal analysis were performed. The obtained spectrum of nanoparticles was compared with the spectrum from the measurement of oleic acid used during the synthesis (Fig. 3). For oleic acid, the signal appearing at 3000 cm⁻¹ is the vibration stretching the C-H bonds in the -C=C-H group, the bands appearing at 2922 cm⁻¹ and 2853 cm⁻¹ correspond to the asymmetrical and symmetrical vibrations stretching the -CH₂ groups, while the intense band at 1710 cm⁻¹ can be attributed to the vibration in the C=O bond. The spectrum measured for magnetic nanoparticles shows the disappearance of the band originating from the C=O bond, while two new bands appear for the wavelengths equal to 1560 cm⁻¹ and 1645 cm⁻¹, corresponding to the asymmetrical and symmetrical stretching vibrations in the -COO- group. The difference in the position of these bands indicates a chelate linkage between the oleic acid -COO- group and iron ions. [Premaratne, W., Priyadarshana, W., Gunawardena, S., De Alwis, A. Synthesis of Nanosilica from Paddy Husk Ash and Their Surface Functionalization. J. Sci. Univ. Kelaniya Sri Lanka 8, 33-48 (2013); Zhang, L., He, R., Gu, H.C. Oleic Acid Coating on the Monodisperse Magnetite Nanoparticles. Appl. Surf. Sci. 253, 2611-2617 (2006); Bronstein, L.M. et al. Influence of Iron Oleate Complex Structure on Iron Oxide Nanoparticle Formation. Chem. Mater. 19, 3624-3632 (2007)].

The crystal structure study of the obtained nanoparticles was carried out on the basis of X-ray powder diffraction (XRD) measurement. The obtained diffractogram was compared with that of microcrystalline magnetite and wüstite (Fig. 4). The lines assigned to the magnetite phase (e.g. for 2Θ≅30°) do not show a shift from the diffraction pattern of the microcrystalline magnetite. However, in the case of wüstite, a shift of the maximum towards higher angles with respect to the reference lines can be observed. This shift suggests a reduction in the crystal lattice constant due to compressive strain occurring in the nanoparticle structure as a result of the core/shell structure. Given the fact that wüstite (FeO) under atmospheric conditions and at even slightly elevated temperatures undergoes rapid oxidation to the magnetite (Fe₃O₄) phase, its oxidized form - maghemite (γ-Fe₂O₃) and hematite (α-Fe₂O₃) or a two-stage disproportionation process leading to the production of hematite and magnetite, it can be concluded that the core of the nanoparticles is wüstite, while the shell is composed of the magnetite or maghemite phase. The strain occurring in the nanoparticle structure may lead to an increase in magnetocrystalline anisotropy. Based on the Scherrer's equation, it was calculated that the average crystallite size of the magnetite/maghemite phase was 4.4 nm, and that of the wüstite phase was 6 nm. Taking into account the nanoparticle structure (core-shell) and using the determined sizes of crystallites, the average size of magnetic nanoparticles can be determined as 14.8 nm. The performed fitting of phases to the obtained diffractogram showed a 22% share of the wüstite phase and a 77% share of magnetite/maghemite [Cornell, R.M., Schwetmann, U. The Iron Oxides: Structure, Properties, Reactions, Occurrences and Uses. 2nd ed. WILEY-VCH GmbH&Co. KGaA, 139-183 (2003); Pichon, B.P. et al. Microstructural and Magnetic Investigations of Wüstite-Spinel Core-Shell Cubic-Shaped Nanoparticles. Chem. Mater. 23, 2886-2900 (2011); Sun, X., Frey Huls, N., Sigdel, A., Sun, S. Tuning Exchange Bias in Core/Shell FeO/Fe3O4 Nanoparticles. Nano Lett. 12, 246-251 (2012); Scherrer, P. Bestimmung der Grösse und der inneren Struktur von Kolloidteilchen mittels Röntgenstrahlen, Nachrichten von der Gesellschaft der Wissenschaften, Göttingen. 98-100 (1918)].

The studies of magnetic nanoparticles with Mössbauer spectroscopy were carried out under various temperature conditions (room temperature, liquid nitrogen temperature and liquid helium temperature). The spectra obtained were compared with the spectra of microcrystalline wüstite and microcrystalline maghemite (Fig. 5). The spectrum measured at room temperature indicates the presence of a magnetically ordered phase through the presence of a broadsextet, while a singlet with an isomeric shift of about 1 mm/s suggests the presence of a second, but paramagnetic phase. The presented spectrum has a relaxational character. This is indicated by a stronger broadening of the lines to the inside and lower amplitudes of the outer lines than the theoretical 3:2:1 to 1:2:3, which means that at room temperature the nanoparticles exhibit superparamagnetic properties. The relaxational spectrum occurs when the frequency of fluctuation of the magnetization vector between the easy magnetization axes is greater than the reciprocal of the ⁵⁷Fe Mossbauer lifetime, i.e. 7 MHz. Fitting of the obtained spectrum to the Blume and Tijon model resulted in the set of parameters presented in Table 1. The value of the isomer shift determined indicates that the paramagnetic phase, represented as singlet in the spectrum, is wüstite. On the other hand, in the case of the spectrum measured at the liquid nitrogen temperature, there is no singlet originating from the paramagnetic phase and the appearance of additional magnetically split component lines indicates the presence of a magnetically ordered phase. The observed lines are much narrower and the intensity ratios are closer to the theoretical 3:2:1 to 1:2:3, which means that at this temperature the nanoparticle magnetization vector cannot overcome the energy barrier and, consequently, no superparamagnetic fluctuations are observed. After subtracting the 22% share of the wüstite phase from the obtained spectrum, it was found that the second iron oxide phase present in the studied sample was maghemite.

The fluctuation frequency of 52 MHz determined on the basis of the relaxation spectrum at room temperature indicates a relatively high superparamagnetic blocking temperature in nanoparticles. On the other hand, the high value of the asymmetry coefficient (ρ=0.95), which corresponds to the relative residence time of the magnetic moment of the nanoparticle along the easy magnetization direction, indicates high magnetocrystalline anisotropy of the obtained systems [Armstrong, R.J., Morrish, A.H. Mössbauer Study Of Ferric Ions In The Tetrahedral And Octahedral Sites Of A Spinel. Phys. Lett. 23, 414-416 (1966); Mørup, S. Mössbauer Effect in Small Iron Particles. Hyperfine Interact. 60, 959-974 (1990); Redl, F.X. et al. Magnetic, Electronic, and Structural Characterization of Nonstoichiometric Iron Oxides at the Nanoscale. J.Am.Chem.Soc. 126, 14583-14599 (2004); Tucek, J., Zboril, R., Petridis, D. Maghemite Nanoparticles by View of Mössbauer Spectroscopy. J. Nanosci. Nanotechnol. 6, 926-947 (2006)].

**Table 1. Parameters obtained after fitting the spectra obtained from Mössbauer spectroscopy measurement of the magnetic nanoparticles and a summary of the literature data for maghemite and wüstite.**

| Measurement temperature | Isomer shift* [mm/s] | Hyperfine field [T] | χ² | Frequency [MHz] | ρ | Intensity [%] |
|---|---|---|---|---|---|---|
| **Magnetic nanoparticles** | | | | | | |
| Room temperature | 0.21(1) | 44.7(2) | 1.1 | 52(4) | 0.95(1) | 55(3) |
| | 0.73(1) | 43.2(2) | | | | 23(1) |
| | 1.05(1) | 0 | | | | 22(2) |
| Liquid nitrogen temperature | 0.30(3) | 49.5(1) | 0.22 | adopted static | - | 61(1) |
| | 0.84(2) | 48.6(3) | | | | 17(3) |
| | 0.99(2) | | | | | 22(2) |

| **Maghemite** | | | | | | |
|---|---|---|---|---|---|---|
| Room temperature | 0.23 | 50.0 | | | | |
| | 0.35 | 50.0 | | | | |
| Liquid helium temperature | 0.40 | 52.0 | | | | |
| | 0.48 | 53.0 | | | | |

| **Wüstite** | | | | | | |
|---|---|---|---|---|---|---|
| Room temperature | 0.95 | | | | | |
| | 0.90 | | | | | |

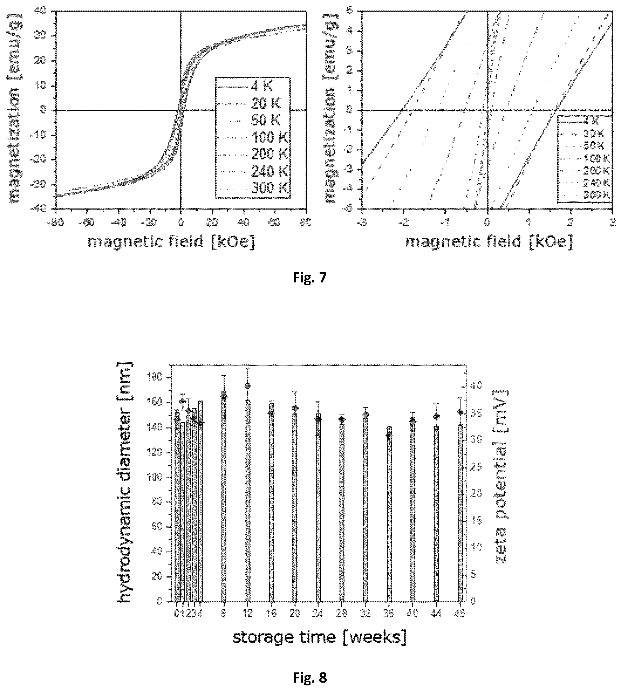

The magnetic nanoparticles were analysed using vibrating sample magnetometry (VSM). Temperature dependence of magnetic susceptibility measured in a static field of 100 Oe in the form of ZFC (zero field cooled) and FC (field cooled) curves (Fig. 6) and magnetization curves as a function of the applied magnetic field for temperatures ranging from 4 to 300 K (Fig. 7) were measured. The temperature at which the magnetic susceptibility value increases significantly (230 K) corresponds to the transition of wüstite from antiferromagnetic to paramagnetic state, i.e. the Neel temperature. Its literature value for wüstite is about 192 K, however, the presence of an additional phase in the form of ferrimagnetic maghemite surrounding the wüstite nanoparticle cores could increase it. The maximum of the ZFC curve allows determination of the average blocking temperature, i.e. the temperature above which the magnetic moments of the nanoparticles exhibit superparamagnetic fluctuations. The blocking temperature value determined on the basis of the measured function is 275 K. The shape of the magnetization curves above this temperature indicates the disappearance of hysteresis, which proves the superparamagnetic state of nanoparticles. During the measurement, the magnetization did not reach saturation, which is related to the presence of wüstite in the nanoparticle structure. The determined values of the coercivity field and remanence are presented in Table 2. Below the Neel temperature, the nanoparticles show a shift of the hysteresis loop as a consequence of the exchange bias coupling at the boundary of the antiferromagnetic wüstite core and the ferrimagnetic maghemite shell, causing the appearance of unidirectional magnetic anisotropy. This coupling is caused by the presence of the antiferromagnetic phase, which is more difficult to remagnetize, and it depends on the size of the core, the thickness of the shell and the shape of the nanoparticle [Pichon, B.P. et al. Microstructural and Magnetic Investigations of Wüstite-Spinel Core-Shell Cubic-Shaped Nanoparticles. Chem. Mater. 23, 2886-2900 (2011); Estrader, M. et al. Origin of the Large Dispersion of Magnetic Properties in Nanostructured Oxides: FexO/Fe3O4 nanoparticles as a Case Study. Nanoscale 7, 3002-3015 (2015)].

**Table 2. Coercivity field and remanence values determined from the magnetization curves of magnetic nanoparticles.**

| | 4 K | 20 K | 50 K | 100 K | 200 K | 240 K | 300 K |
|---|---|---|---|---|---|---|---|
| Coercivity field (+-) [kOe] | -2.05 | -1.80 | -1.22 | -0.55 | -0.11 | -0.01 | -0.00 |
| | 1.64 | 1.59 | 1.08 | 0.45 | 0.09 | 0.01 | 0.00 |
| Remanence [emu/g] | 6.4 | 7.1 | 5.7 | 3.1 | 1.5 | 0.2 | 0.0 |

### Example 2. Preparation of cationic capsules based on liquid cores with encapsulated magnetic nanoparticles.

The capsules were obtained in the emulsification process, and a hydrophobically and cationically modified chitosan derivative, an anionic chitosan derivative and magnetic nanoparticles obtained by thermal decomposition of an organometallic precursor were used for their preparation.

The preparation procedure for the liquid oil core capsules based on the phenomenon of self-assembly of amphiphilic graft polymers was previously described by Szafraniec et al. [Szafraniec, J. et al. Chitosan-based nanocapsules of core-shell architecture. Polimery 62, 509-515 (2017); Szafraniec, J., Janik, M., Odrobińska, J., Zapotoczny, S. Nanocapsules templated on liquid cores stabilized by graft amphiphilic polyelectrolytes. *Nanoscale* **7,** 5525-5536 (2015)]. Due to the additional encapsulation of magnetic nanoparticles, the above-mentioned procedure has been modified. Thus, 10 µL of oleic acid with dispersed 100 mg/mL magnetic nanoparticles were added to 1 mL of 0.15 M sodium chloride (NaCl) solution with 1 mg of dissolved cationically and hydrophobically modified chitosan (CChitC12). CChitC12 was obtained according to the procedure described earlier (Karewicz A., Bielska D., Loboda A. et al.: Colloids and Surfaces B: Biointerfaces 2013, 109, 307. http://dx.doi.org/10.1016/j.colsurfb.2013.03.059). Diagram below, m:n:p = 67.5:2:30.5.

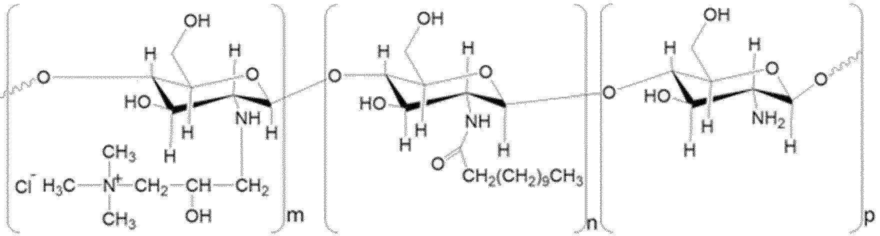

The obtained solution was subjected to vigorous mixing in a shaker, and then impulse sonication (in the mode of 1 s work, 2 s pause, room temperature) in order to accurately deposit the polymer layer on the surface of the oil capsule cores. The non-encapsulated magnetic nanoparticles were removed by magnetic separation. The resulting emulsion showed a greyish-white colour.

### Physicochemical characteristics of the cationic capsules

Dynamic light scattering (DLS) technique was used to carry out measurements for determination of the hydrodynamic diameter size and the value of the zeta potential. The measurements were repeated for 48 weeks, during which time the test samples were stored at 4°C. The hydrodynamic diameter and zeta potential values of capsules with positive surface charge remained at the same level throughout the entire period of the study (Fig. 8). The average hydrodynamic diameter value for these systems did not exceed 170 nm, nor was it lower than 140 nm, with the polydispersity index lower than 0.3, which proves a relatively narrow size distribution. On the other hand, the value of the zeta potential was within the range of 30-40 mV throughout the entire measurement period.

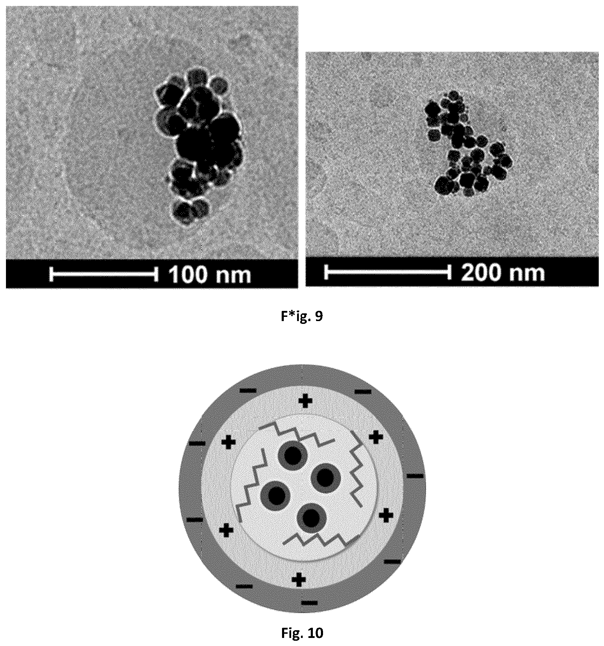

Cationic magnetic capsules were imaged using cryogenic transmission electron microscopy (cryoTEM) (Fig. 9). The obtained images confirm the presence of magnetic nanoparticles inside the carrier, and the spherical shape of the carrier can also be observed with a size consistent with DLS measurements.

### Example 3. Preparation of anionic capsules based on liquid cores with encapsulated magnetic nanoparticles.

The second type of capsules studied were systems having a negative surface charge (Fig. 10). The anionic capsules were prepared based on the technique of applying oppositely charged layers. In order to create such carriers, 0.6 mL of 1 g/L anionic chitosan solution (AChit, prepared according to the procedure described earlier: Bulwan M., Zapotoczny S., Nowakowska M.: Soft Matter 2009, 5, 4726. http://dx.doi.org/10.1039/B909355A diagram below, degree of sulfonic group substitution 49 mol%) dissolved in 0.0015 M sodium chloride was added to 0.4 mL of cationic capsule dispersion. The obtained solution was subjected to 10-minute vigorous mixing on a shaker.

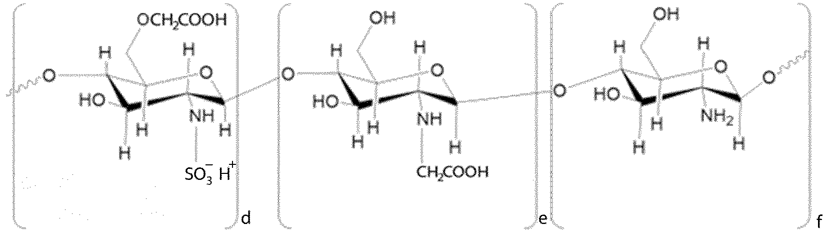

### Physicochemical characteristics of anionic capsules

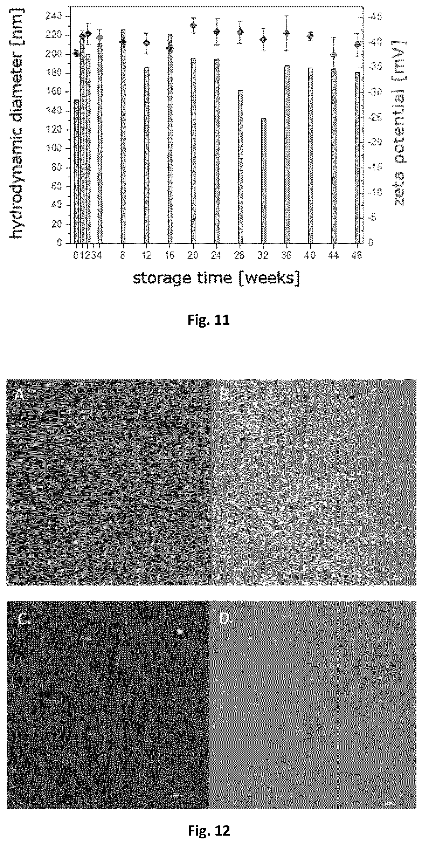

Measurements of the hydrodynamic diameters and the zeta potential values performed for the anionic capsules are shown in Fig. 11. The measured mean values of the hydrodynamic diameters were in the range of 140-230 nm, with a polydispersity index less than 0.4. The larger size distribution may be related to the formation of a small amount of aggregates or to the non-homogeneous coating of the capsules by anionic chitosan (AChit). The zeta potential values range from -35 to -45 mV. Both the higher mean values of the hydrodynamic diameters in comparison to the cationic capsules and the negative value of the zeta potentials indicate the correct deposition of the anionic chitosan layer on the surface of the analysed carriers.

### Example 4. Preparation of capsules based on liquid fluorescent cores with encapsulated magnetic nanoparticles.

For the sake of confocal microscope imaging, fluorescent modification of the studied capsules was necessary. The preparation procedures for both cationic and anionic capsules have been extended by a step involving the addition of a fluorescent dye to the oil suspension of magnetic nanoparticles which later form the core of the carrier. In the case of capsules enriched with perylene dye, 1 mg of dye was added per 1 mL of oil mixture containing dispersed magnetic nanoparticles.

Imaging of the capsules additionally containing a hydrophobic fluorescent dye in the form of perylene in the oil core was performed with confocal microscopy (Fig. 12). The presented images show the blue fluorescent glow of the labelled oil capsule cores. The obtained images confirm the possibility of placing hydrophobic substances, in particular hydrophobic active substances inside the magnetic capsule cores, moreover, images obtained two weeks after the capsule preparation confirm the feasibility of a longer encapsulation of substances soluble in the hydrophobic environment.

### Example 5. Preparation of concentrated dispersions of cationic capsules based on liquid cores with encapsulated magnetic nanoparticles.

Vibrating-sample magnetometry (VSM) measurement was performed to verify magnetic properties of the capsules. In order to obtain the appropriate signal-to-noise ratio, it was necessary to prepare a capsule suspension containing an increased concentration of magnetic nanoparticles, and hence an increased amount of carrier suspended in the solution. The sample preparation was performed according to the procedure analogous to the cationic capsule formation procedure using 30 µL of oleic acid with dispersed magnetic nanoparticles at a concentration of 100 g/L and 1 mL of chitosan derivative (CChitC12) at a concentration of 10 g/L of 0.15 M NaCl solution.

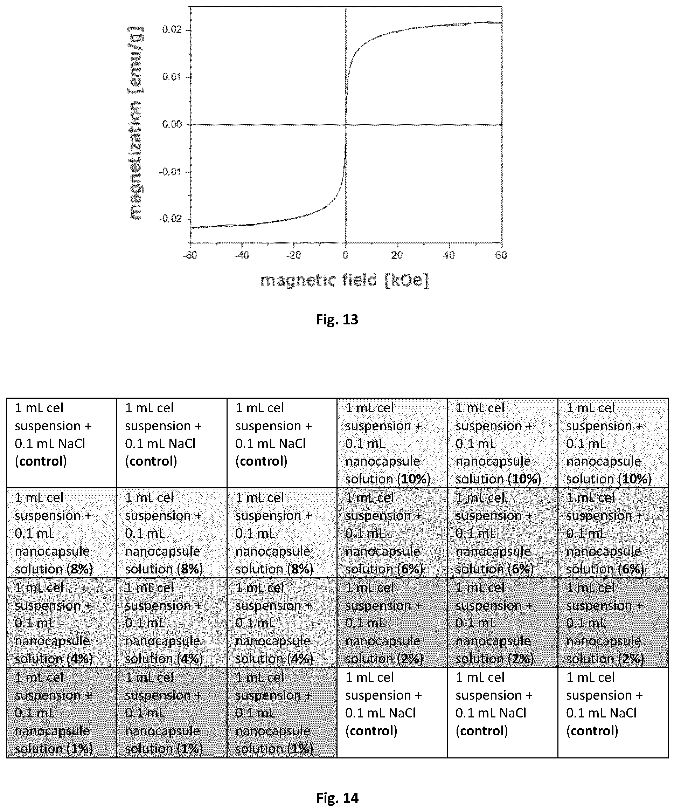

The curves resulting from the magnetometric measurements confirm the magnetic nature of the carriers, while the absence of hysteresis loops is related to the presence of superparamagnetic nanoparticles in the capsule core (Fig. 13).

### Example 6. Experimental studies involving cell lines.

*In vitro* studies were performed using the 4T1 line, which is a cancer cell line derived from murine mammary gland tissue. The culture was maintained under sterile conditions at 37°C and in the presence of 5% CO₂. Cell culture was carried out using a medium supplemented with an antibiotic in the form of penicillin and streptomycin solution (1%) and fetal bovine serum (5%). All reagents used in the cell studies were heated in a water bath at 37°C. In the first step, cells were thawed for culture, the cell suspension was transferred to a centrifuge tube, and 5 mL of medium was added to dilute the dimethylsulfoxide (DMSO). The cells were then centrifuged (1000 rpm, 5 min), the solution overlaying the cell pellet decanted and cells resuspended in 1 mL of medium. The cells thus prepared were seeded onto a culture dish previously filled with 10 mL of medium. The proliferation of cells and their condition during culture were monitored with an optical microscope, and the medium was replaced every 2 days. In order to carry out a passage, trypsinization was performed. The dish with the expanded cells was rinsed with PBS solution twice. Subsequently 0.8 mL of trypsin solution was added, which was drawn off and again 0.8 mL of trypsin was added. The treated dish was placed in the incubator and after 3 minutes it was tapped thoroughly and put back into the incubator. After another 3 minutes, the dish was tapped again, and the degree of detachment of the cells from the substrate was monitored under the microscope. The detached cells were transferred from the dish to a falcon tube following addition of 3 mL of medium for trypsin inactivation. The obtained cell suspension was centrifuged (1000 rpm, 5 minutes), the fluid poured off, and the pelleted cells were suspended in 1 mL of medium. Thereby, a cell suspension was obtained, which was used in the subsequent experiments.

### Example 7. Cytotoxicity study of capsules against the 4T1 line cancer cells (XTT).

The XTT assay was performed to determine the cytotoxicity of the cationic and anionic capsules against the 4T1 line. This assay allows to determine the metabolic activity of cellular mitochondria based on a spectrophotometric measurement of XTT tetrazole salts reduction to formazan due to mitochondrial dehydrogenase activity. Two 24-well plates containing cells of the 4T1 cell line and the appropriate dispersion concentrations of the cationic capsules (plate 1) and the anionic capsules (plate 2) were prepared the day before direct determination of capsule toxicity to the selected cell line. Plates were prepared by adding 0.9 mL of medium and then 100 µL of cell suspension to each well. Each well was mixed thoroughly by pipetting and shaking the plate several times. The seeded plates were placed in an incubator for 4 hours. The next step was adding the agent in the form of capsules. For this, fluid was aspirated from each well of the plate and 1 mL of medium was added. Then 0.1 mL of the agent was added to each well, which was the capsules diluted in 0.15 M NaCl (for cationic capsules) or 0.0015 M NaCl (for anionic capsules) reaching the concentrations shown in Fig. 14 and 0.15 M NaCl (for cationic capsules) or 0.0015 M NaCl (for anionic capsules) solution alone, the obtained solutions were mixed in the wells by pipetting. Prepared plates were placed in the incubator for 24 hours.

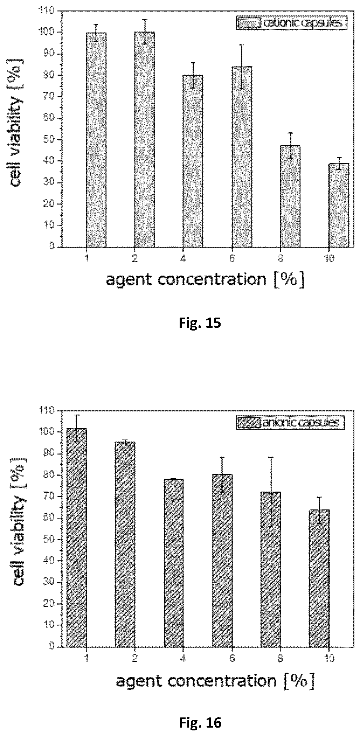

Prior to the experiment, the reagents necessary for the XTT assay were prepared. Reagents were preheated to 37°C according to the manufacturer's instructions and 5 mL of XTT labeling reagent and 0.1 mL of the coupling reagent were mixed. Before adding the solution, the plates prepared the day before were removed from the incubator and the liquid in them was pipetted out, adding 0.2 mL of fresh medium and then 0.1 mL of the XTT solution into each well. The plates were gently mixed and placed in the incubator. After 1 hour, the spectrophotometric measurement of the absorption was performed. The obtained results presented in the graph (Figs. 15 and 16) clearly show the relationship between the decreasing concentration of the agent and the subsequent increase in cell survival. On the basis of the obtained values it can be concluded that the capsules having a negative surface charge are less toxic to the cells. However, both in the case of anionic and cationic capsules, it can be seen that the agent concentration of 2% did not induce a negative response in the assayed cells.

### Example 8. Directing capsules into cancer cells using a static magnetic field.

The possibility of magnetic control of capsules containing magnetic nanoparticles was verified by conducting an experiment using a static, non-uniform magnetic field applied to a cuvette filled with capsules suspended in a medium and a microscope slide containing live cells on its surface. In order to exclude the influence of gravitational forces on the penetration of capsules into the cells, the slide with the cell culture was placed in a vertical position.

Before starting the main experiment, it was necessary to create slide cultures, for this purpose properly cut and sterile microscopic coverslips were used and placed in 6-well cell culture plates. Then, a cell culture slide was placed on the sidewall of a sterile cuvette and 1 mL of medium and 0.1 mL of a 2% capsule dispersion in 0.15 M NaCl (for cationic capsules) and 0.0015 M NaCl (for anionic capsules) were poured therein.

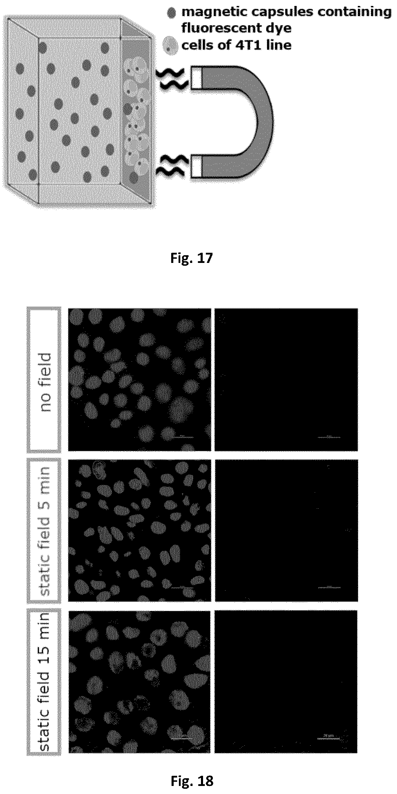

The cuvettes thus prepared were subjected to an external static magnetic field (for 5 min and 15 min) according to the scheme presented in Fig. 17. The distance between the sample and the magnet was 1 cm, which corresponded to a magnetic induction of 143 mT. Also, experiments were performed to verify spontaneous penetration of capsules into the cells and the influence of the external static magnetic field on the condition of the cells.

Once the experiment was completed, the cells on the slide were fixed, so they could be subsequently imaged using a confocal microscope. For fixation, the cell slides were transferred to a 6-well plate and washed twice with 2 mL of PBS solution. Then 2 mL of 10% formalin solution in PBS was added to each well. After 10 minutes, the solution was removed, the slides were rinsed with PBS solution and placed on a microscopic glass slide, protecting the edges with varnish.

Capsules additionally enriched with a fluorescent probe in the form of the Nile Red dye were used for the experiment. The procedure for creating such capsules consisted in adding the dye, at a concentration of 5 mg/mL, to the oil suspension of the magnetic nanoparticles before starting the addition of chitosan (CChitC12) dissolved in water to the solution.

Cell viability was also verified after conducting experiments with the external magnetic field. For this purpose, two solutions were prepared, 10 mg of Hoechst dissolved in 1 mL of deionized water and diluted with PBS solution in a volume ratio of 1:2000 and 1 mg of propidium iodide dissolved in 1 mL of deionized water and diluted in PBS solution in a volume ratio of 1:3000. Then, the cells on the slide fixed after the external magnetic field experiment, were incubated with the respective dye for 5 minutes, rinsed 3 times with PBS solution, placed on a microscopic glass slide and protected with varnish. The specimens thus obtained were imaged with a confocal microscope.

### Example 9. Verification of the influence of a static magnetic field on the condition of cells for cationic capsules and anionic capsules.

In the first stage of the study, the influence of external static magnetic field on cancer cells of the 4T1 line was evaluated with no carrier present. The collected images (Fig. 18) definitely exclude a negative influence of the static magnetic field, which is evidenced by the lack of fluorescent signal in cells stained with propidium iodide, which penetrates only through damaged cell membranes.

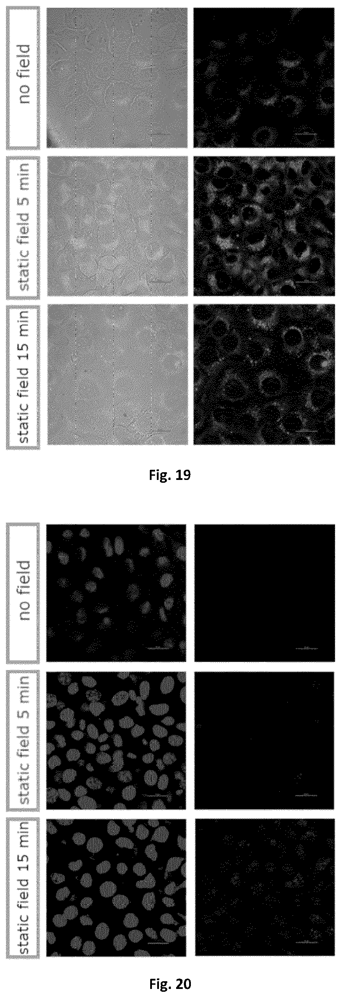

Then, studies were carried out to verify the possibility of navigating the cationic capsules into the cancer cells with an external static magnetic field. The presented images (Fig. 19) show a slight tendency for spontaneous penetration of cationic capsules, while the use of the external static magnetic field increases the effectiveness of the studied phenomenon, and primarily contributes to the accumulation of capsules within the vicinity of cancer cells.

The effectiveness of the cationic capsule penetration process assisted by a static external magnetic field was verified by fluorescent staining of cells after the experiment. The obtained images (Fig. 20) confirm that a 15-minute exposure to the static magnetic field in the presence of magnetic cationic capsules significantly contributes to a break in the integrity of the cell membrane.

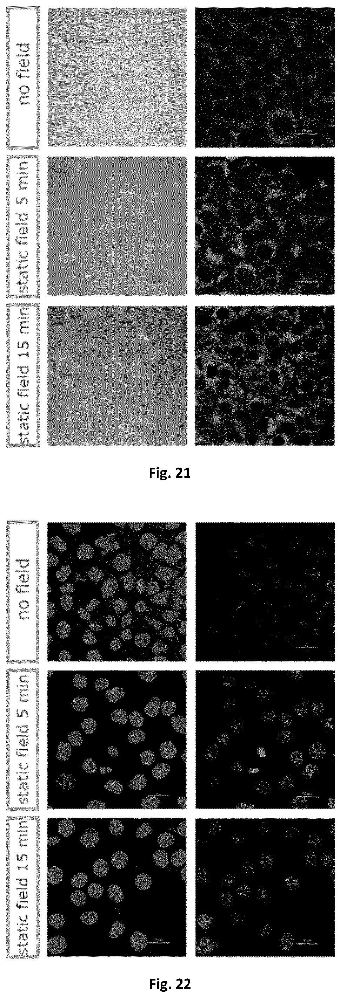

An analogous experiment of carrier navigation using an external magnetic field was carried out for the anionic capsules. The obtained images (Fig. 21) indicate the possibility of spontaneous penetration of capsules with a negative surface charge inside the cells by endocytosis, but the use of the external static magnetic field increases the effectiveness of the penetration process.

Fluorescence labelling of cells exposed to a static external magnetic field in the presence of anionic capsules and without the external field was performed and is shown in Fig. 22. The obtained images confirm the increased tendency of spontaneous penetration of the anionic capsules inside the cells. Visible bright red glow after the application of the external static magnetic field indicates a significant break in the integrity of the cell membrane, confirming the successful introduction of the carrier into the cancer cells.

### Example 10. Alternating magnetic field-assisted release of the encapsulated substance from capsules placed inside cancer cells.

The possibility of releasing the substance encapsulated in the carrier was evaluated by applying an alternating magnetic field. The use of the external alternating magnetic field was performed as a continuation of the static magnetic field experiment. The above-described system, after being subjected to a 15-minute exposure to a static magnetic field, was transferred for 5 minutes to an external alternating magnetic field with a frequency of 50 Hz and various induction values, as shown in Table 3.

**Table 3. The magnetic induction values in experiments with alternating magnetic field.**

| Field | Induction value [mT] |
|---|---|
| Weak | 22 |
| Medium | 67 |
| Strong | 222 |

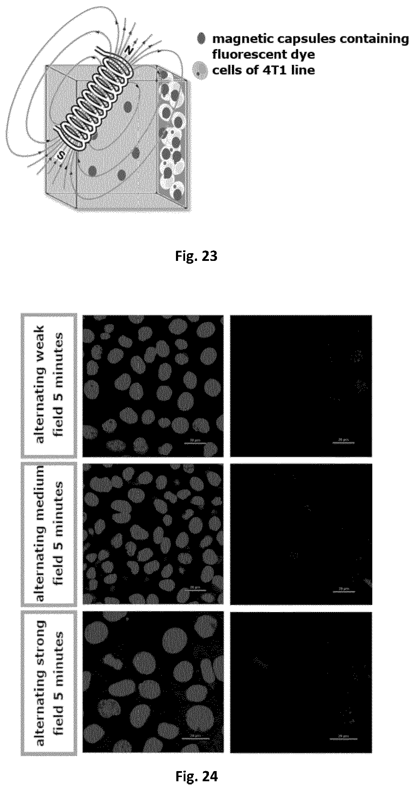

Experiments were also carried out to exclude the harmful effects of the alternating field to cells, by carrying out the so-called blank experiments on cells with no contact with the capsules. The diagram of the experimental procedure is shown in Fig. 23. Once the experiment was completed, the slides with the cells were fixed according to the previously described protocol. A verification of the cell viability after the experiment was also carried out, analogous to the one described earlier, with the use of Hoechst dyes and propidium iodide.

### Example 11. Verification of the influence of an alternating magnetic field on the condition of cells.

The results of the experiment allowing to verify the harmful effect of the alternating magnetic field on cancer cells are presented in the confocal microscope images (Fig. 24). The obtained images confirm the maintained integrity of the cell membrane after exposure of cells to an external alternating magnetic field, which is indicated by the lack of fluorescence in cells stained with propidium iodide. The results of the experiment with a 15-minute exposure of fluorescently-labelled cationic capsules and cancer cells to an external static magnetic field and a 5-minute exposure to an alternating magnetic field with various parameters are shown in Fig. 25. The obtained images indicate that in the case of cationic capsules, the strong alternating magnetic field applied contributes to a significant crossing of the cell membrane barrier, but does not lead to an effective release of the encapsulated substance inside the cell.

The break in the cell membrane integrity is confirmed by images of cells fluorescently stained after the experiment involving exposure to a static external magnetic field and an alternating magnetic field of cells in the presence of cationic capsules (Fig. 26). The images obtained indicate the possibility of introducing the capsules inside the cells with damaging the cell membrane, which will contribute to their death.

Verification of the possibility of targeted and magnetically-controlled release of the encapsulated substance inside the cells was also carried out for capsules with a negative surface charge. The images shown (Fig. 27) were obtained after a 15-minute exposure to a static magnetic field and a 5-minute exposure to a respective alternating magnetic field of the cells surrounded by the anionic capsules containing a fluorescent dye. The results obtained for the sample exposed to a strong alternating magnetic field clearly confirm the possibility of an effective release of the encapsulated substance inside the cancer cells.

The results of the evaluation of the cell membrane after the experiment aimed at magnetically-controlled introduction and release of the substance carried within the anionic capsule are shown in Fig. 28. Bright red fluorescent glow confirms significant damage of the cell membrane, especially in the case of applying a strong alternating magnetic field. These damages affect all cells in the image that were subjected to experiment using a strong alternating magnetic field and ultimately lead to the death of cancer cells.

### List of reagents used:

1) chitosan (molar mass: 50-190 kDa, Sigma-Aldrich),
2) glycidyl trimethyl ammonium chloride (GTMAC, ≥90%, Sigma-Aldrich),
3) N-dodecyl aldehyde (92%, Sigma-Aldrich),
4) sodium cyanoborohydride (NaCNBH3, 95%, Sigma-Aldrich),
5) carboxymethyl chitosan (CMC, deacetylation degree 90%, AK Scientific),
6) sulfur trioxide-trimethylamine complex (TMST, 99%, Sigma-Aldrich),
7) sodium bicarbonate (NaHCO₃, p.a., Sigma-Aldrich),
8) sodium hydroxide (NaOH, p.a., Avantor Performance Materials Poland S.A.),
9) iron (III) chloride (FeCl₃, anhydrous, Merck),
10) sodium oleate (> 97%, TCI),
11) octadecane (90%, Alfa Aesar),
12) oleic acid (OA, 99.5%, Alfa Aesar),
13) perylene (Pe, gold label, 99.9%, Sigma-Aldrich),
14) Nile Red (NR, for microscopy, Sigma-Aldrich),
15) n-octadecane (p.a., Polyscience Corp.),
16) Dulbecco's Modified Eagle Medium (DMEM, high-glucose, Sigma-Aldrich),
17) Fetal Bovine Serum (FBS, HyClone),
18) Penicillin-Streptomycin solution (Symbios),
19) trypsin (HyClone),
20) a mixture of salts for the preparation of a phosphate buffer (PBS, tablet, Sigma-Aldrich),
21) dimethylsulfoxide (DMSO, ≥99.7%, Sigma-Aldrich),
22) XTT (cell proliferation kit II, Sigma-Aldrich),
23) formalin (36.5-38% aqueous solution, Sigma-Aldrich),
24) Hoechst (Sigma-Aldrich),
25) propidium iodide (PI, Sigma-Aldrich),
26) acetic acid (99.5%, Chempur),
27) sodium chloride (NaCl, p.a., Lachner),
28) ethanol (96%, Chempur),
29) acetone (p.a., Chempur),
30) methanol (p.a., Chempur),
31) hexane (p.a., Chempur),
32) cellulose dialysis tubes (14,000 g/mol cut-off, Sigma Aldrich),
33) inert gas - argon,
34) all cell assays were carried out on the breast cancer cell line derived from the mammary gland tissue of the murine BALB/c strain (line 4T1, ATCC CRL-2539),
35) all aqueous solutions were prepared with deionized water.

## Claims

1. Magnetic core/shell polymer nanocapsule containing iron oxide nanoparticles and a chitosan derivative, **characterized in that** it consists of a liquid oil core in which magnetic iron oxide nanoparticles are suspended, preferably wüstite crystallites and crystallites of the magnetitemaghemite phase, preferably with a percentage ratio of 22:77 respectively, provided with an inner shell containing a cationic chitosan derivative and a negatively-charged outer shell containing an anionic chitosan derivative, the oil core containing a known hydrophobic active substance or dye.

2. The nanocapsule of claim 1, **characterized in that** the concentration of the iron oxide nanoparticles in the oil core is at least 100 g/L.

3. The nanocapsule of claim 1, **characterized in that** the dye is perylene or nile red, and the active substance is a known hydrophobic anticancer drug, preferably selected from: paclitaxel, lapatinib, fulvestrant or a mixture thereof.

4. The nanocapsule of claim 1, **characterized in that** the oil core comprises oleic acid.

5. The nanocapsule of claim 1, **characterized in that** the cationic chitosan derivative comprises N-[(2-hydroxo-3-trimethylamino)propyl]chitosan chloride or n-dodecyl hydrophobic groups.

6. The nanocapsule of claim 1, **characterized in that** the anionic chitosan derivative comprises carboxymethyl chitosan.

7. The nanocapsule of claim 1, **characterized in that** it disintegrates under the influence of an alternating magnetic field with a frequency of 50 Hz, a magnetic induction of not less than 222 mT, and a field duration time of at least 5 min.

8. The nanocapsule of claim 1, **characterized in that** the size of the iron oxide nanoparticles is 15-30 nm.

9. The nanocapsule of claim 1, **characterized in that** the wüstite crystallite size is not larger than 6 nm.

10. The nanocapsule of claim 1, **characterized in that** the crystallite size of the magnetitemaghemite phase is not larger than 4.4 nm.

11. The nanocapsule of claim 1, **characterized in that** the hydrodynamic size of the polymer nanocapsules is 140-230 nm.

12. The nanocapsule of claim 1, **characterized in that** in the anionic system the dispersion coefficient of the polymer nanocapsules is not greater than 0.4.

13. The nanocapsule of claim 1, **characterized in that** the zeta potential value of the polymer nanocapsules in the anionic system is from -35 to -45 mV.

14. A pharmaceutical composition containing magnetic polymer nanocapsules as defined in claims 1-13.

15. A magnetic polymer nanocapsule as defined in claims 1-13 for use in pharmacy or diagnostics, especially for use in the treatment or prophylaxis of cancer, in particular of breast cancer.

## Patentansprüche

1. Magnetische, eine(n) Kern/Hülle aufweisende Polymer-Nanokapsel, die Eisenoxid-Nanopartikel und ein Chitosanderivat enthält, **dadurch gekennzeichnet, dass** sie aus Folgendem besteht: einem flüssigen Ölkern, in dem magnetische Eisenoxid-Nanopartikel suspendiert sind, vorzugsweise Wüstit-Kristallite und Kristallite der Magnetit-Maghemit-Phase, vorzugsweise mit einem Prozentverhältnis von - entsprechend - 22 : 77, und der mit einer inneren Hülle, die ein kationisches Chitosanderivat enthält, und einer negativ geladenen äußeren Hülle, die ein anionisches Chitosanderivat enthält, versehen ist, wobei der Ölkern einen bekannten hydrophoben Wirkstoff oder Farbstoff enthält.

2. Nanokapsel nach Anspruch 1, **dadurch gekennzeichnet, dass** die Konzentration der Eisenoxid-Nanopartikel in dem Ölkern mindestens 100 g/l beträgt.

3. Nanokapsel nach Anspruch 1, **dadurch gekennzeichnet, dass** der Farbstoff Perylen oder Nilrot ist und der Wirkstoff ein bekanntes hydrophobes Krebsarzneimittel ist, das vorzugsweise aus folgenden ausgewählt ist: Paclitaxel, Lapatinib, Fulvestrant oder einem Gemisch davon.

4. Nanokapsel nach Anspruch 1, **dadurch gekennzeichnet, dass** der Ölkern Ölsäure umfasst.

5. Nanokapsel nach Anspruch 1, **dadurch gekennzeichnet, dass** das kationische Chitosanderivat N-[(2-hydroxo-3-trimethylamino)propyl]chitosanchlorid oder hydrophobe n-Dodecyl-Gruppen umfasst.

6. Nanokapsel nach Anspruch 1, **dadurch gekennzeichnet, dass** das anionische Chitosanderivat Carboxymethylchitosan umfasst.

7. Nanokapsel nach Anspruch 1, **dadurch gekennzeichnet, dass** sie unter dem Einfluss eines magnetischen Wechselfelds mit einer Frequenz von 50 Hz, einer magnetischen Induktion von wenigstens 222 mT und einer Felddauer von mindestens 5 min zerfällt.

8. Nanokapsel nach Anspruch 1, **dadurch gekennzeichnet, dass** die Größe der Eisenoxid-Nanopartikel 15 - 30 nm beträgt.

9. Nanokapsel nach Anspruch 1, **dadurch gekennzeichnet, dass** die Größe der Wüstit-Kristallite nicht größer als 6 nm ist.

10. Nanokapsel nach Anspruch 1, **dadurch gekennzeichnet, dass** die Kristallitgröße der Magnetit-Maghemit-Phase nicht größer als 4,4 nm ist.

11. Nanokapsel nach Anspruch 1, **dadurch gekennzeichnet, dass** die hydrodynamische Größe der Polymer-Nanokapseln 140 - 230 nm beträgt.

12. Nanokapsel nach Anspruch 1, **dadurch gekennzeichnet, dass** in dem anionischen System der Dispersionskoeffizient der Polymer-Nanokapseln höchstens 0,4 beträgt.

13. Nanokapsel nach Anspruch 1, **dadurch gekennzeichnet, dass** der Wert des Zeta-Potentials der Polymer-Nanokapseln in dem anionischen System -35 bis -45 mV beträgt.

14. Pharmazeutische Zusammensetzung, die magnetische Polymer-Nanokapseln, wie in den Ansprüchen 1 - 13 definiert, enthält.

15. Magnetische Polymer-Nanokapsel, wie in den Ansprüchen 1 - 13 definiert, zur pharmazeutischen oder diagnostischen Verwendung, insbesondere zur Verwendung in der Behandlung oder Prophylaxe von Krebs, insbesondere von Brustkrebs.

## Revendications

1. Nanocapsule polymère à noyau/enveloppe magnétique contenant des nanoparticules d'oxyde de fer et un dérivé de chitosane, **caractérisée en ce qu'**elle est constituée d'un noyau huileux liquide dans lequel sont en suspension des nanoparticules magnétiques d'oxyde de fer, de préférence des cristallites de wüstite et des cristallites de la phase magnétite-maghémite, de préférence dans un rapport en pourcentage de 22:77 respectivement, pourvues d'une enveloppe interne contenant un dérivé cationique de chitosane et d'une enveloppe externe chargée négativement contenant un dérivé anionique de chitosane, le noyau huileux contenant une substance active ou un colorant hydrophobe connu.

2. Nanocapsule selon la revendication 1, **caractérisée en ce que** la concentration des nanoparticules d'oxyde de fer dans le noyau huileux est d'au moins 100 g/L.

3. Nanocapsule selon la revendication 1, **caractérisée en ce que** le colorant est le pérylène ou le rouge nile, et la substance active est un médicament anticancéreux hydrophobe connu, de préférence choisi parmi le paclitaxel, le lapatinib, le fulvestrant ou un mélange de ceux-ci.

4. Nanocapsule selon la revendication 1, **caractérisée en ce que** le noyau huileux comprend de l'acide oléique.

5. Nanocapsule selon la revendication 1, **caractérisée en ce que** le dérivé cationique du chitosane comprend du chlorure de N-[(2-hydroxo-3-triméthylamino)propyl]chitosane ou des groupes hydrophobes n-dodécyliques.

6. Nanocapsule selon la revendication 1, **caractérisée en ce que** le dérivé anionique du chitosane comprend du carboxyméthylchitosane.

7. Nanocapsule selon la revendication 1, **caractérisée en ce qu'**elle se désintègre sous l'influence d'un champ magnétique alternatif ayant une fréquence de 50 Hz, une induction magnétique d'au moins 222 mT et une durée de champ d'au moins 5 minutes.

8. Nanocapsule selon la revendication 1, **caractérisée en ce que** la taille des nanoparticules d'oxyde de fer est comprise entre 15 et 30 nm.

9. Nanocapsule selon la revendication 1, **caractérisée en ce que** la taille des cristallites de wüstite n'est pas supérieure à 6 nm.

10. Nanocapsule selon la revendication 1, **caractérisée en ce que** la taille des cristallites de la phase magnétite-maghémite n'est pas supérieure à 4,4 nm.

11. Nanocapsule selon la revendication 1, **caractérisée en ce que** la taille hydrodynamique des nanocapsules de polymère est comprise entre 140 et 230 nm.

12. Nanocapsule selon la revendication 1, **caractérisée en ce que**, dans le système anionique, le coefficient de dispersion des nanocapsules de polymère n'est pas supérieur à 0,4.

13. Nanocapsule selon la revendication 1, **caractérisée en ce que** la valeur du potentiel zêta des nanocapsules de polymère dans le système anionique est comprise entre -35 et -45 mV.

14. Composition pharmaceutique contenant des nanocapsules de polymère magnétique telles que définies dans les revendications 1 à 13.

15. Nanocapsule de polymère magnétique telle que définie dans les revendications 1 à 13, destinée à être utilisée en pharmacie ou en diagnostic, en particulier pour le traitement ou la prophylaxie du cancer, en particulier du cancer du sein.
